# DEMANDE DE BREVET EUROPEEN

(11) **EP 1 247 518 A1**
(43) Date de publication de la demande: **09.10.2002**
(21) Numéro de dépôt: 02290687.9
(22) Date de dépôt: 19.03.2002
(51) Int. Cl.: A61K 7/09, A61K 7/00

(54) **Procédé de déformation permanente des cheveux mettant en oeuvre des silicones aminées**

(30) Priorité: 06.04.2001 FR 0104727
(71) Demandeur: L'OREAL, 75008 Paris (FR)
(72) Inventeur: Devin-Baudoin, Priscille, 92170 Vanves (FR); Sabbagh, Anne, 92500 Rueil-Malmaison (FR)
(74) Mandataire: Bourdeau, Françoise

(57) **Abrégé**

L'invention concerne un procédé de déformation permanente des fibres kératiniques comprenant l'application sur les fibres kératiniques, avant l'opération de réduction et/ou après l'opération de fixation, d'une composition cosmétique de pré-traitement et/ou de post-traitement comprenant, dans un véhicule cosmétiquement acceptable, une microémulsion de silicone aminée, la taille primaire moyenne en nombre des particules de la microémulsion étant comprise entre 3 et 70 nm.

## Description

La présente invention concerne un procédé déformation permanente des fibres kératiniques, en particulier des cheveux, ledit procédé étant notamment utilisable dans les salons de coiffure, professionnels ou par des particuliers, via la commercialisation de kits.

Au sens de la présente invention, on entend par « *procédé de déformation permanente »,* tout procédé durable dans le temps, de mise en forme des cheveux, de frisage, de défrisage ou de décrêpage.

On entend par *« fibres kératiniques »*, en particulier, les cheveux, les cils, les sourcils, et surtout les cheveux.

On sait que la technique la plus usuelle pour obtenir une déformation permanente des cheveux consiste, dans un premier temps, à réaliser l'ouverture des liaisons disulfures -S-S- de la kératine (cystine) à l'aide d'une composition réductrice, contenant un agent réducteur (étape de réduction) puis, après avoir de préférence, rincé la chevelure ainsi traitée, à reconstituer dans un second temps lesdites liaisons disulfures en appliquant, sur les cheveux préalablement mis sous tension (bigoudis et autres), une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est éminemment durable dans le temps et résiste notamment à l'action des lavages à l'eau ou des shampooings, et ceci par opposition aux simples techniques classiques de déformation temporaire, telles que de mise en plis.

Les compositions réductrices utilisables pour la mise en oeuvre de 1a première étape d'une opération de permanente contiennent généralement, à titre d'agents réducteurs, des sulfites, des bisulfites ou des thiols. Parmi ces derniers, on peut citer la cystéïne et ses divers dérivés, la cystéamine et ses dérivés, l'acide thiolactique, l'acide thioglycolique ainsi que ses esters, notamment le monothioglycolate de glycérol, et le thioglycérol.

Concernant les compositions oxydantes nécessaires à la mise en oeuvre de l'étape de fixation, on fait le plus souvent appel, dans la pratique, à des compositions à base d'eau oxygénée ou de bromates alcalins.

Le problème des techniques de permanentes connues à ce jour est que leur application répétée sur les cheveux induit à la longue une altération progressive de la qualité du cheveu, et notamment une altération progressive et marquée de la brillance et des propriétés cosmétiques du cheveu, en particulier au niveau de la douceur des fibres qui ont tendance à devenir de plus en plus rêches ainsi qu'au niveau de leur démêlage, les cheveux devenant de plus en plus difficiles à démêler. Cette altération est en outre particulièrement accentuée lorsque l'étape de fixation de l'opération de déformation permanente est effectuée à l'aide d'un bromate.

Pour limiter cette altération des cheveux, il a déjà été proposé d'introduire directement, dans la composition réductrice, des agents de conditionnement. Par exemple, les demandes de brevet japonais H2-250814 et H9-151120 décrivent des compositions réductrices contenant des silicones aminées, pouvant éventuellement se présenter sous la forme de microémulsion.

Cependant, les procédés de déformation permanente des cheveux utilisant de telles compositions ne donnent pas encore entière satisfaction, dans la mesure où le degré, la qualité et la nervosité de frisure sont généralement insuffisants et éphémères, comme si l'agent de conditionnement, notamment les silicones aminées, directement associé à l'agent réducteur, freinaient l'activité de ce dernier.

Le problème posé par l'invention est de fournir un procédé de déformation permanente des fibres kératiniques, notamment des cheveux, qui réduise la dégradation mécanique et/ou cosmétique des cheveux, tout en apportant un degré, une qualité et une nervosité de frisure satisfaisants.

La Demanderesse a découvert, de manière surprenante et inattendue, qu'en appliquant sur les cheveux, avant d'appliquer la composition réductrice et/ou après avoir appliqué la composition oxydante, au moins une composition cosmétique de pré-traitement et/ou de post-traitement contenant une micoémulsion de silicone aminée, il était possible de résoudre le problème posé par la présente invention.

L'invention a pour objet un procédé de déformation permanente des fibres kératiniques, notamment des cheveux, comprenant au moins les opérations consistant à :
(i) appliquer sur les fibres kératiniques une composition réductrice ;
(ii) réaliser l'oxydation des fibres kératiniques,
caractérisé par le fait qu'il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, une microémulsion de silicone aminée, la taille primaire moyenne en nombre des particules de la microémulsion étant comprise entre 3 et 70 nm.

Un autre objet de l'invention concerne un kit pour la déformation permanente des fibres kératiniques, l'un des compartiments contenant une composition cosmétique de pré-traitement et/ou de post-traitement comprenant une microémulsion de silicone aminée, la taille primaire moyenne en nombre des particules de la microémulsion étant comprise entre 3 et 70 nm.

### SILICONE AMINEE EN MICROEMULSION

Au sens de la présente invention, on entend par « *microémulsion de silicone aminée »*, des dispersions thermodynamiquement stables constituées d'une phase discontinue formée par des particules de silicones aminés dans une phase continue formée par de l'eau,associée ou non à des tensioactifs.

On entend par *« taille primaire »*, la dimension maximale qu'il est possible de mesurer entre deux points diamétralement opposés des particules.

De préférence, la taille primaire moyenne en nombre des particules de la microémulsion est comprise entre 5 et 60 nm, et plus préférentiellement encore entre 10 et 50 nm.

Dans tout ce qui suit ou qui précède, on entend désigner par silicone ou polysiloxane, en conformité avec l'acceptation générale, tout polymère ou oligomère organosilicié à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements amides, les radicaux acyloxy ou acyloxyalkyls, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon l'invention, on désigne par silicone aminée toute silicone comportant au moins une amine primaire, secondaire, tertiaire ou un groupement ammonium quaternaire. On peut ainsi citer :
(a) les polysiloxanes répondant à la formule : 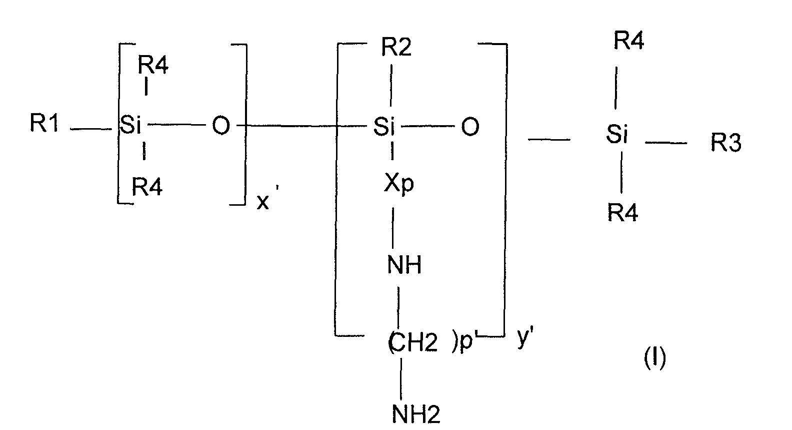
   dans laquelle x' et y' sont des nombres entiers dépendant du poids moléculaire, généralement tels que ledit poids moléculaire moyen en poids est compris entre 5 000 et 500 000 environ ;
   R1, R2, R3 identiques ou différents désignent un radical hydroxyle, un radical alkyle en C1 à C4, un radical alcoxy en C1 à C4, phényle ;
   X désigne un radical alkylène en C1-C4, ramifié ou non ;
   R4 désigne un radical alkyle en C1-C4 ou phényle ;
   p, p' désignent indépendamment l'un de l'autre un nombre entier variant de 1 à 10.
   De préférence, alkyle désigne méthyl et alcoxy désigne méthoxy.
   De préférence, p=3, p'=2 et R4 désigne méthyl et X désigne méthylène.
   Parmi ces polymères, on peut citer les composés désignés par l'appellation « amodiméthicone » et « triméthylsilylaminodiméthicone » dans le dictionnaire CTFA.
(b) les silicones aminées répondant à la formule : 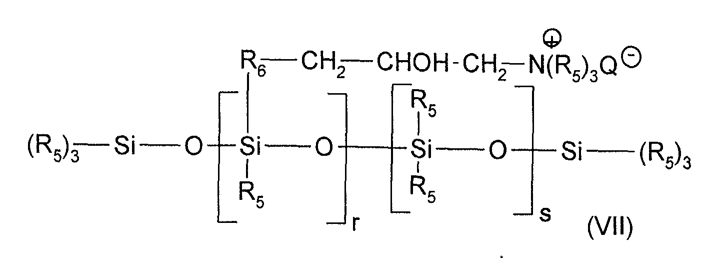 dans laquelle
   R₅ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   Q⁻ est un anion tel qu'un ion halogénure, notamment chlorure ou un sel d'acide organique (acétate ...);
   r représente une valeur statistique moyenne de 2 à 20 et en particulier de 2 à 8 ;
   s représente une valeur statistique moyenne de 20 à 200 et en particulier de 20 à 50.
   De telles silicones aminées sont décrites plus particulièrement dans le brevet US 4 185 087.
   Une silicone entrant dans cette classe est la silicone commercialisée par la société Union Carbide sous la dénomination "Ucar Silicone ALE 56.
c) les silicones ammonium quaternaire de formule : 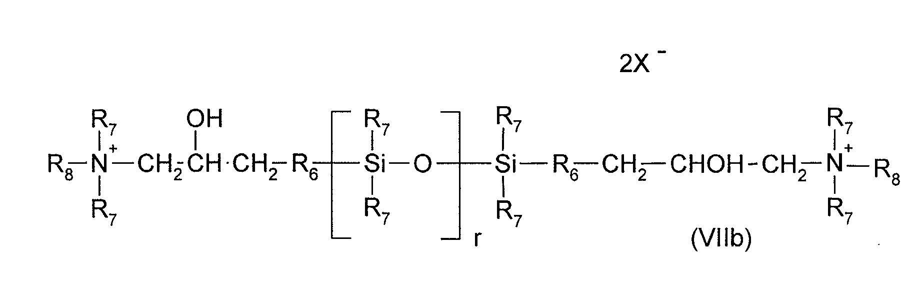 dans laquelle
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈ , un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;

Ces silicones sont par exemple décrites dans la demande EP-A-0530974.

Selon l'invention, on préfère utiliser une silicone aminée:
- dont l'indice d'amine est supérieur à 0,15 meq par gramme,
- possédant des extréminés hydroxy et/ou alcoxy.

A titre d'exemple, on peut citer, comme microémulsion, contenant une telle silicone, le produit commercialisé par la Société WACKER sous le nom de WACKER FINISH CT96E.

Avantageusement, la concentration en silicone aminée dans la composition de pré-traitement ou de post-traitement est comprise entre 0,05 et 10 % en poids par rapport au poids total de cette composition, et plus avantageusement entre 0,1,et 7 %.

Les microémulsions conformes à la présente invention peuvent contenir des solvants.

Ces solvants sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.

Les solvants tels que ceux cités ci-dessus sont présents dans les microémulsions de l'invention dans des concentrations allant, de préférence, de 0,01 à 30% en poids par rapport au poids total de l'émulsion.

En outre, l'utilisation des alcools tels que définis ci-dessus, à des concentrations supérieures ou égales à 5% en poids et de préférence supérieure à 15%, permet d'obtenir des microémulsions sans conservateur.

Les compositions de pré-traitement ou de post-traitement contenant les microémulsions utilisées selon l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes et des parfums.

De préférence, le pH de la composition de pré-traitement ou de post-traitement comprenant une microémulsion de silicone aminée est compris de préférence entre 2 et 10, et plus préférentiellement entre 3 et 9.

La composition de pré-traitement comprenant une microémulsion de silicone aminée est appliquée sur les cheveux à traiter, lesquels auront été, éventuellement, préalablement mouillés. Cette application peut être réalisée après l'habituelle étape de mise sous tension des cheveux sous une forme correspondant à la forme finale désirée pour ces derniers (boucles par exemple), cette étape pouvant, elle-même, être mise en oeuvre par tout moyen, mécanique notamment, approprié et connu en soi pour maintenir sous tension des cheveux, tels que par exemple rouleaux, bigoudis et analogues.

Avantageusement, on laisse agir, à température ambiante ou sous chaleur, sur les cheveux la composition de pré-traitement ou de post-traitement comprenant une microémulsion de silicone aminée pendant une durée comprise entre 1 et 60 minutes, et plus avantageusement entre 3 et 30 minutes.

Selon une étape facultative du procédé, on peut rincer les cheveux imprégnés de la composition de pré-traitement comprenant une microémulsion de silicone aminée, le rinçage étant effectué généralement à l'eau.

Dans une étape indispensable du procédé selon l'invention, on applique sur les cheveux une composition réductrice, ladite composition réductrice contenant généralement au moins un thiol.

Le thiol de la composition réductrice peut être choisi parmi les thiols connus comme agents réducteurs tels que par exemple l'acide thioglycolique, le monothioglycolate de glycérol ou de glycol, la cystéamine et ses dérivés acylés en C₁-C₄ tels que la N-acétyl cystéamine ou la N-propionyl cystéamine, la cystéine, la N-acétylcystéine, les N-mercaptoalkylamides de sucres tels que le N-(mercapto-2-éthyl)gluconamide, l'acide 3-mercaptopropionique et ses dérivés, l'acide thiolactique et ses esters tel que le monothiolactate de glycérol, l'acide thiomalique, la panthétéine, le thioglycérol, les sulfites ou les bisulfites d'un métal alcalin ou alcalino-terreux, les N-(mercaptoalkyl)ω-hydroxyalkylamides décrits dans la demande de brevet EP-A-354.835 et les N-mono- ou N,N-dialkylmercapto 4-butyramides décrits dans la demande de brevet EP-A-368.763, les aminomercaptoalkylamides décrits dans la demande de brevet EP-A-432.000, les dérivés des acides N-(mercaptoalkyl) succinamiques ou des N-(mercaptoalkyl) succiminides décrits dans la demande de brevet EP-A-465.342, les alkylaminomercaptoalkylamides décrits dans la demande de brevet EP-A-514.282, le mélange de thioglycolate d'hydroxy-2-propyle et de thioglycolate d'hydroxy-2-méthyl-1 éthyle décrit dans la demande de brevet FR-A-2.679.448.

On utilise de préférence l'acide thioglycolique, l'acide thiolactique et l'acide 3-mercaptopropionique.

Les agents réducteurs sont généralement présents à une concentration qui peut être comprise entre 1 et 20 % en poids par rapport au poids total de la compositon réductrice.

Le pH de la composition réductrice est, généralement, compris entre 6 et 10 et de préférence entre 7 et 9.

Les pH des compositions réductrices peuvent être ajustés clasiquement par ajout d'agents basifiants, tels que par exemple l'ammoniaque, la monoéthanolamine, la diéthanolamine, la triéthanolamine, l'isopropanolamine, la propanediamine-1,3, un carbonate ou bicarbonate de métal alcalin ou d'ammonium, un carbonate ou bicarbonate d'amines primaires, secondaires ou tertiaires ou un carbonate organique tel que le carbonate de guanidine, tous ces composés pouvant bien entendu être pris seuls ou en mélange.

La composition réductrice peut se présenter sous la forme d'une lotion, épaissie ou non, d'une crème, d'un gel ou de toute autre forme appropriée et peut contenir des additifs connus pour leur utilisation dans les compositions réductrices pour la déformation permanente des cheveux.

La composition réductrice peut être également du type exothermique, c'est-à-dire provoquant un certain échauffement lors de l'application sur les cheveux, ce qui apporte un agrément à la personne qui subit la permanente ou le défrisage.

La composition réductrice peut également contenir un solvant tel que par exemple de l'éthanol, du propanol ou de l'isopropanol ou encore du glycérol à une concentration maximale de 20 % par rapport au poids total de la composition.

Lorsque les compositions sont destinées à une opération de défrisage ou de décrêpage des cheveux, la composition réductrice est de préférence sous forme d'une crème épaissie de façon à maintenir les cheveux aussi raides que possible. On réalise ces crèmes, sous forme d'émulsions "lourdes", par exemple à base de stéarate de glycéryle, de stéarate de glycol, de cires auto-émulsionnables, d'alcools gras, etc.

On peut également utiliser des liquides ou des gels contenant des agents épaississants tels que des polymères ou des copolymères carboxyvinyliques qui "collent" les cheveux et les maintiennent dans la position lisse pendant le temps de pose.

Enfin, les compositions peuvent être également sous forme dite "auto-neutralisante" ou encore "auto-régulée" et dans ce cas, les agents réducteurs utilisés selon l'invention sont associés à au moins un disulfure connu pour son utilisation dans une composition réductrice pour permanente auto-neutralisante.

Il convient, de manière classique, de laisser reposer pendant quelques minutes, généralement entre 2 et 40 minutes, de préférence entre 5 et 30 minutes, la chevelure sur laquelle a été appliquée la composition réductrice, et ceci de façon à bien laisser le temps au réducteur d'agir correctement sur les cheveux. Cette phase d'attente est effectuée généralement en laissant reposer à l'air libre (à température ambiante ou avec chauffage) la chevelure traitée. Pendant cette phase d'attente, on prend généralement soin que les cheveux ne sèchent pas complètement et restent humides.

Les cheveux imprégnés de la composition réductrice peuvent être, ensuite, rincés soigneusement, généralement à l'eau. Eventuellement, après rinçage, on met en oeuvre une phase de chauffage à température élevée pendant quelques secondes.

Puis, on applique sur les cheveux ainsi rincés une composition oxydante dans le but de fixer la nouvelle forme imposée aux cheveux. Il peut également être envisagé de laisser les cheveux s'oxyder à l'air.

La composition oxydante contient un agent oxydant qui peut être choisi parmi l'eau oxygénée, un bromate alcalin, un persel ou un polythionate. Comme indiqué précédemment, l'un des grands avantages du procédé selon l'invention est qu'il convient parfaitement dans le cas des compositions oxydantes à base de bromates. La concentration en bromates dans la composition oxydante est généralement comprise entre 0,1 et 2 M.

Le pH de la composition oxydante est généralement compris entre 2 et 10.

Comme dans le cas de l'application de la composition réductrice, la chevelure sur laquelle a été appliquée la composition oxydante est ensuite, de manière classique, laissée dans une phase de repos ou d'attente qui dure quelques minutes, généralement entre 3 et 30 minutes, de préférence entre 5 et 15 minutes.

La composition de post-traitement comprenant une microémulsion de silicone aminée est, de préférence, appliquée après rinçage de la composition oxydante, sur des cheveux humides ou secs. Les cheveux ayant subi le post-traitement peuvent éventuellement être séchés et/ou chauffées et/ou rincés, avant le coiffage. Le cas échéant, la composition peut être appliquée alors que les cheveux sont maintenus par un dispositif mécanique, comme des rouleaux de mise en pli ou des bigoudis.

Le plus souvent, les cheveux imprégnés de la composition oxydante sont rincés soigneusement, généralement à l'eau. On sépare, avant ou après le rinçage, la fibre kératinique des moyens nécessaire à la mise sous tension.

On obtient finalement une chevelure présentant de bonnes propriétés cosmétiques : les cheveux sont plus brillants, plus doux et plus faciles à démêler ou à coiffer.

De manière avantageuse, la composition de pré-traitement ou de post-traitement contenant une microémulsion de silicone aminée est appliquée selon l'une au moins des variantes suivantes :
- sur des cheveux propres et humides, avant la mise en oeuvre des moyens de mise sous tension des cheveux, sans rincer les cheveux avant l'application du réducteur ;
- sur des cheveux humides après rinçage du fixateur, les cheveux étant ultérieurement soit rincés, soit séchés.

Lorsque le procédé de déformation permanente des cheveux est un procédé de défrisage, on peut, de manière connue en soi, mettre en oeuvre, un défrisant généralement soit thiolé, soit alcalin.

Dans le cas de défrisant thiolé, le procédé conforme à l'invention est avantageusement mis en oeuvre en appliquant la composition de pré-traitement ou de post-traitement contenant une microémulsion de silicone aminée selon l'une au moins des variantes suivantes :
- sur des cheveux propres et humides, sans rinçage avant l'application du réducteur ;
- sur des cheveux propres et humides, après le rinçage du fixateur, en rinçant avant le séchage des cheveux.

Dans le cas de défrisant alcalin, le procédé conforme à l'invention est avantageusement mis en oeuvre en appliquant la composition de pré-traitement ou de post-traitement contenant une microémulsion de silicone aminée sur cheveux humides, après rinçage de shampooing neutralisant, en rinçant avant le séchage des cheveux.

Dans le cas d'un frisage des cheveux, le procédé conforme à l'invention procure des boucles nerveuses et les cheveux sont spécialement souples, légers, soyeux et bien individualisés.

Dans le cas d'un défrisage des cheveux, le procédé conforme à l'invention procure notamment une maîtrise du volume de la chevelure, un lissage des cheveux de la racine à la pointe et un toucher plus naturel.

L'invention pourra être mieux comprise à l'aide des exemples non limitatifs qui suivent et qui constituent des modes de mise en oeuvre avantageux du procédé selon l'invention.

m.a. signifie matière active.

### EXEMPLES :

### Exemple 1 :

On utilise une composition de pré-traitement contenant la microémulsion de silicone aminée à 2 % de m.a. en final en poids WACKER FINISH CT96E (WACKER), la taille primaire moyenne en nombre des particules étant de 20 nm. On applique cette composition sur une mèche de cheveux moyennement sensibilisés, propres et humides. La mèche est passée sous chaleur, à 60 °C, pendant 15 minutes, puis rincée. Elle subit ensuite un traitement avec la permanente Dulcia Vital 2 ® pour cheveux sensibilisés, commercialisée par L'Oréal. Le même traitement de permanente est appliqué parallèlement sur une mèche non traitée avec la composition de pré-traitement (art antérieur). Les mesures de solubilité alcaline et de porosité sont rassemblées dans le tableau 1.

**Tableau 1**

| | Solubilité alcaline | Porosité |
|---|---|---|
| Procédé selon l'invention | 22 % | 55 |
| Procédé selon l'art antérieur | 26 % | 59 |

On constate que le pré-traitement conforme à la présente invention a pour effet de diminuer la dégradation des cheveux, après l'action de la permanente.

Pour mesurer la solubilité alcaline (SA), on procède comme suit : Les cheveux à analyser sont traités avec une solution de soudeN/10, chauffés à 65 °C pendant 30 minutes. La solubilité alcaline est le pourcentage de masse de cheveux perdus lors de ce traitement.

Pour mesurer la porosité, on procède comme suit : Les cheveux à analyser sont placés dans une solution contenant un colorant non chargé (2-nitroparaphénylènediamine), essorés puis désorbés dans deux solutions aqueuses tamponnées. L'absorbance A de ces solutions de désorption réunies est mesurée à 470 nm. La porosité est obtenue par la relation : 100xA-20

Exemple 2 : On utilise une lotion de pré-traitement contenant 2 % en matière active de silicone WACKER FINISH CT96E. La lotion est appliquée sur des cheveux humides, maintenue en forme par des bigoudis, un bonnet est posé. La chevelure est passée sous un casque chauffant pendant 5 minutes. Le bonnet est ôté et la chevelure est rincée. Le traitement de permanente est ensuite appliqué de façon classique, avec Dulcia Vital 2. Ce procédé est appliqué à six personnes, ayant les cheveux naturels, sur unr demi-tête en comparaison avec une permanente Dulcia Vital 2 a permis d'obtenir une amélioratopn de la douceur, de la nervosité de la frisure, du gonflabnt et du toucher. Au fur et à mesure des shampooings, les différences cosmétiques se maintiennent pendant environ six semaines.

Exemple 3 : On utilise une lotion de pré-traitement contenant 2 % en matière active de silicone WACKER FINISH CT96E. La chevelure est laissée 15 minutes sous chaleur. Une crème défrisante à l'acide thiolactique STIFF est ensuite appliquée. Le reste du défrisage est effectué de façon classique.
Ce procédé appliqué à cinq personnes sur une demi tête en comparaison avec le défrisant STIFF a permis d'avoir une amélioration de la douceur, du démêlage, de la facilité de coiffage et une meilleure maîtrise du volume. Après plusieurs shampooings, la chevelure traitée avec la composition est plus douce et moins gonflante.

Exemple 4 : On utilise une lotion de pré-traitement contenant 2 % en matière active de silicone WACKER FINISH CT96E, sur une chevelure humide ayant préalablement reçu un traitement de défrisage Goldys sans soude. La chevelure ainsi traitée est laissée 10 minutes à la température ambiante, la composition est ensuite rincée.
Ce procédé, appliqué à 11 personnes, ayant des cheveux naturels crépus, sur une demi tête, en comparaison avec le défrisant Goldys sans soude, a permis d'obtenir une amélioration de la douceur, du démêlage et un bon contrôle du volume. après chaque shampooing, le côté traité par la composition de silicone aminé est plus doux et facile à démêler.

Exemple 5 : On utilise une lotion de pré-traitement contenant 2 % en matière active de silicone WACKER FINISH CT96E, après le rinçage du fixateur. La chevelure est mise en forme à l'aide d'un brushing, sans rinçage de cette composition.
Ce procédé, appliqué à cinq personnes ayant des cheveux très sensibilisés sur une demi tête en comparaison avec une permanente classique a permis d'avoir une amélioration du démêlage, de la douceur, de la nervosité de la frisure, du toucher et du gonflant le jour de l'application. Pendant les six semaines qui suivent cette permanente, la frisure disparaît plus rapidement du côté non traité avec la silicone. La demi tête traitée avec la microémulsion de silicone reste toujours nettement plus douce et plus facile à démêler, les cheveux sont plus faciles à coiffer et la chevelure est plus gonflante.

## Revendications

1. Procédé de déformation permanente des fibres kératiniques, notamment des cheveux, comprenant au moins les opérations consistant à :
(i) appliquer sur les fibres kératiniques une composition réductrice ;
(ii) réaliser l'oxydation des fibres kératiniques,
**caractérisé par le fait qu'**il comprend, en outre, l'application sur les fibres kératiniques, avant l'opération (i) et/ou après l'opération (ii), d'une composition cosmétique de pré-traitement et/ou de post-traitement comprenant, dans un véhicule cosmétiquement acceptable, une microémulsion de silicone aminée, la taille primaire moyenne en nombre des particules de la microémulsion étant comprise entre 3 et 70 nm.

2. Procédé selon la revendication 1, **caractérisé par le fait que** la taille primaire moyenne en nombre des particules de la microémulsion est comprise entre 5 et 60 nm, et plus préférentiellement encore entre 10 et 50 nm.

3. Procédé selon l'une quelconque des revendications précédentes, **caractérisé par le fait que** le pH de la composition de pré-traitement ou de post-traitement comprenant la microémulsion de silicone aminée est compris de préférence entre 2 et 10, et plus préférentiellement entre 3 et 50.

4. Procédé selon l'une quelconque des revendications précédentes, **caractérisé par le fait que** la concentration en silicone aminée dans la composition de pré-traitement ou de post-traitement est comprise entre 0,05 et 10 % en poids par rapport au poids total de cette composition, et plus avantageusement entre 0,1,et 7 %.

5. Procédé selon l'une quelconque des revendications précédentes, **caractérisé par le fait qu'**on laisse agir sur les cheveux la composition de pré-traitement ou de post-traitement comprenant la microémulsion de silicone aminée pendant une durée comprise entre 1 et 60 minutes, et plus avantageusement entre 3 et 30 minutes.

6. Procédé selon l'une quelconque des revendications précédentes, **caractérisé par le fait que** la silicone aminée répond à la formule I : 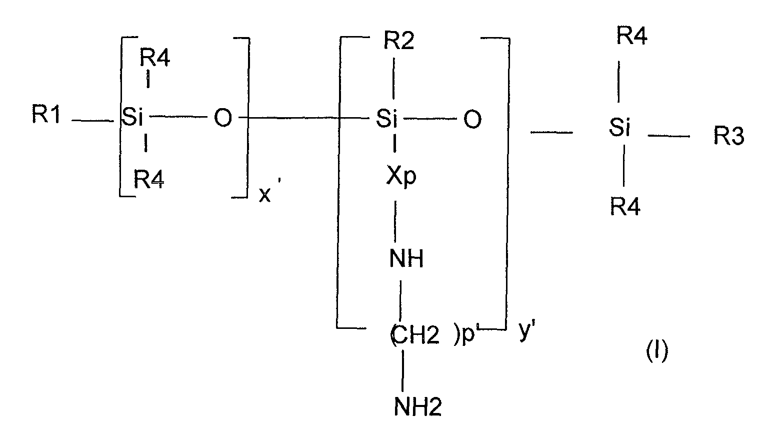 dans laquelle x' et y' sont des nombres entiers dépendant du poids moléculaire, généralement tels que ledit poids moléculaire moyen en poids est compris entre 5 000 et 500 000 environ ;
R1, R2, R3 identiques ou différents désignent un radical hydroxyle, un radical alkyle en C1 à C4, un radical alcoxy en C1 à C4, phényle ;
X désigne un radical alkylène en C1-C4, ramifié ou non ;
R4 désigne un radical alkyle en C1-C4 ou phényle ;
p, p' désignent indépendamment l'un de l'autre un nombre entier variant de 1 à 10.

7. Procédé selon l'une quelconque des revendications précédentes, **caractérisé par le fait qu'**on utilise une silicone aminée dont l'indice d'amine est supérieur à 0,15 meq par gramme.

8. Procédé selon l'une quelconque des revendications précédentes, **caractérisé par le fait qu'**on utilise une silicone aminée possédant des extréminés hydroxy et/ou alcoxy.

9. Procédé selon l'une quelconque des revendications précédentes, **caractérisée par le fait que** la composition de pré-traitement ou de post-traitement comprend, en outre, des additifs choisis parmi les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les huiles essentielles, les humectants, les filtres solaires siliconés ou non, des agents conservateurs, des séquestrants, des agents nacrants, des pigments, des agents hydratants, des agents antipelliculaires, des agents antiséborrhéiques, des plastifiants, des hydroxyacides, des électrolytes et des parfums.

10. Kit pour la déformation permanente des fibres kératiniques, l'un des compartiments contenant une composition cosmétique de pré-traitement et/ou de post-traitement comprenant une microémulsion de silicone aminée, la taille moyenne en nombre des particules de la microémulsion étant comprise entre 3 et 70 nm.

11. Utilisation d'une composition cosmétique de pré-traitement et/ou de post-traitement comprenant, dans un véhicule cosmétiquement acceptable, une microémulsion de silicone aminée, 1a taille moyenne en nombre des particules de la microémulsion étant comprise entre 3 et 70 nm, pour la déformation permanente des fibres kératiniques.
